# EUROPEAN PATENT SPECIFICATION

(11) **EP 2 918 680 B1**
(45) Date of publication and mention of the grant of the patent: **03.04.2019**
(21) Application number: 13853496.1
(22) Date of filing: 26.04.2013
(51) Int. Cl.: C12N 15/15, C12N 15/63, C12N 15/82, A01H 5/10, C07K 14/81, C07K 1/18

(54) **METHOD FOR PRODUCING, ISOLATING AND PURIFYING RECOMBINANT HUMAN ANTITRYPTASE (OSRAAT) FROM RICE SEEDS**
VERFAHREN ZUR HERSTELLUNG, ISOLIERUNG UND REINIGUNG VON REKOMBINANTER HUMANER ANTITRYPTASE (OSRAAT) AUS REISSAMEN
PROCÉDÉ POUR PRODUIRE, ISOLER ET PURIFIER DE L'ANTITRYPTASE HUMAINE RECOMBINANTE (OSRAAT) À PARTIR DE SEMENCES DE RIZ

(30) Priority: 07.11.2012 CN 201210441102
(43) Date of publication of application: 16.09.2015
(73) Proprietor: WUHAN HEALTHGEN BIOTECHNOLOGY CORP, East Lake High-Tech Development Zone Wuhan, Hubei 430079 (CN)
(72) Inventor: YANG, Daichang, Wuhan Hubei 430079 (CN); SHI, Qianni, Wuhan Hubei 430079 (CN); ZHANG, Liping, Wuhan Hubei 430079 (CN)
(74) Representative: Høiberg P/S
(86) International application number: PCT/CN2013/000482
(87) International publication number: WO 2014/071681

(56) References cited:
- WO-A1-2004/099405
- WO-A1-2005/017168
- WO-A1-2011/121031
- WO-A1-2012/083580
- WO-A2-02/064750
- WO-A2-02/064814
- AU-A1- 2007 216 827
- CN-A- 1 073 717
- CN-A- 1 896 239
- CN-A- 102 994 514
- LNNERDAL: "Expression of human milk proteins in plants.", JOURNAL OF THE AMERICAN COLLEGE OF NUTRITION, vol. 21, no. 3 Suppl, 1 June 2002 (2002-06-01), pages 218S-221S, XP055018949, ISSN: 0731-5724
- KAREN A. MCDONALD ET AL: "Production of Human [alpha]-1-Antitrypsin from Transgenic Rice Cell Culture in a Membrane Bioreactor", BIOTECHNOLOGY PROGRESS., vol. 21, no. 3, 1 January 2005 (2005-01-01), pages 728-734, XP055258350, US ISSN: 8756-7938, DOI: 10.1021/bp0496676
- Y. HE ET AL: "Large-scale production of functional human serum albumin from transgenic rice seeds", PROCEEDINGS OF THE NATIONAL ACADEMY OF SCIENCES, vol. 108, no. 47, 31 October 2011 (2011-10-31), pages 19078-19083, XP055258351, US ISSN: 0027-8424, DOI: 10.1073/pnas.1109736108
- Elizabeth E Murrayl2 ET AL: "Codon usage in plant genes", Nucleic Acids Research, vol. 17, no. 2 25 January 1989 (1989-01-25), XP055258357, Retrieved from the Internet: URL:http://www.ncbi.nlm.nih.gov/pmc/articl es/PMC331598/pdf/nar00211-0013.pdf [retrieved on 2016-03-15]
- ZHANG LIPING ET AL: "Expression and characterization of recombinant human alpha-antitrypsin in transgenic rice seed", JOURNAL OF BIOTECHNOLOGY, ELSEVIER SCIENCE PUBLISHERS, AMSTERDAM, NL, vol. 164, no. 2, 31 January 2013 (2013-01-31), pages 300-308, XP028990894, ISSN: 0168-1656, DOI: 10.1016/J.JBIOTEC.2013.01.008 Retrieved from the Internet: URL:http://ac.els-cdn.com/S016816561300017 5/1-s2.0-S0168165613000175-main.pdf?_tid=1 2614bd0-eaa8-11e5-b2c1-00000aab0f27&acdnat =1458044508_a3099a43a0115bd9a8c6a2219b10a8 ff> [retrieved on 2016-03-14]
- RAINER BISCHOFF ET AL: "Purification and biochemical characterization of recombinant .alpha.1-antitrypsin variants expressed in Escherichia coli", BIOCHEMISTRY, vol. 30, no. 14, 1 April 1991 (1991-04-01) , pages 3464-3472, XP055048825, ISSN: 0006-2960, DOI: 10.1021/bi00228a017
- DATABASE GENBANK [Online] 05 November 2011 'Homo sapiens serpin peptidase inhibitor, clade A (alpha-1 antiproteinase, antitrypsin), member 1 (SERPINA1), transcript variant 3, mRNA', XP055255370 Retrieved from NCBI Database accession no. NM_001002235
- HUANG, JIANMIN ET AL.: 'Expression and Purification of Functional Human r-1-Antitrypsin from Cultured Plant Cells.' BIOTECHNOL. PROG. vol. 17, no. 1, 2001, pages 126 - 133, XP002367520

### SEQUENCE LISTING

<110> HEALTHGEN BIOTECHNOLOGY CO., LTD.
<120> METHOD FOR PRODUCING, ISOLATING AND PURIFYING RECOMBINANT HUMAN ANTITRYPTASE (OsrAAT) FROM RICE SEEDS
<130> 13P420046
<160> 6
<170> PatentIn version 3.5
<210> 1
   <211> 1185
   <212> DNA
   <213> Artificial Sequence
<220>
   <221> misc_feature
   <222> (1) .. (1185)
   <223> Codon-optimized human AAT gene (OsrAAT Sequence)
<400> 1
   gaggacccgc agggcgacgc cgcccagaag accgacacca gccaccacga ccaggaccac 60
   ccgaccttca acaagatcac cccgaacctc gccgagttcg ccttcagcct ctaccgccag 120
   ctcgcccacc agagcaacag caccaacatc ttcttcagcc cggtgagcat cgccaccgcc 180
   ttcgccatgc tcagcctcgg caccaaggcc gacacccacg acgagatcct ggagggcctc 240
   aacttcaacc tcaccgagat cccggaggcc cagatccacg agggcttcca ggagctcctc 300
   cgcaccctca accagccgga cagccagctc cagctcacca ccggcaacgg cctcttcctc 360
   agcgagggcc tcaagctcgt ggacaagttc ctggaggacg tgaagaagct ctaccacagc 420
   gaggccttca ccgtgaactt cggcgacacc gaggaggcca agaagcagat caacgactac 480
   gtggagaagg gcacccaggg caagatcgtg gacctcgtga aggagctcga ccgcgacacc 540
   gtgttcgccc tcgtgaacta catcttcttc aagggcaagt gggagcgccc gttcgaggtg 600
   aaggacaccg aggaggagga cttccacgtg gaccaggtga ccaccgtgaa ggtgccgatg 660
   atgaagcgcc tcggcatgtt caacatccag cactgcaaga agctcagcag ctgggtgctc 720
   ctcatgaagt acctcggcaa cgccaccgcc atcttcttcc tcccggacga gggcaagctc 780
   cagcacctgg agaacgagct cacccacgac atcatcacca agttcctgga gaacgaggac 840
   cgccgcagcg ccagcctcca cctcccgaag ctcagcatca ccggcaccta cgacctcaag 900
   agcgtgctcg gccagctcgg catcaccaag gtgttcagca acggcgccga cctcagcggc 960
   gtgaccgagg aggccccgct caagctcagc aaggccgtgc acaaggccgt gctcaccatc 1020
   gacgagaagg gcaccgaggc cgccggcgcc atgttcctgg aggccatccc ggtgagcatc 1080
   ccgccggagg tcaagttcaa caagccgttc gtgttcctca tgatcgagca gaacaccaag 1140
   agcccgctct tcatgggcaa ggtcgtcaac ccgacccaga agtga 1185
<210> 2
   <211> 2832
   <212> DNA
   <213> Artificial Sequence
<220>
   <221> misc_feature
   <223> Expression cassette sequence containing Gtl3a promoter and signal
   peptide, the codon-optimized AAT gene and Nos terminator
<400> 2
   ttttctctta ggtttacccg ccaatatatc ctgtcaaaca ctgatagttt aaactgaagg 60
   cgggaaacga caatctgatc atgagcggag aattaaggga gtcacgttat gacccccgcc 120
   gatgacgcgg gacaagccgt tttacgtttg gaactgacag aaccgcaacg ttgaaggagc 180
   cactcagccg cgggttgtgg agctccaccg cggtggcggc cgctctagaa ctagtggatc 240
   ccccctggcg aaagggggat gtgctgcaag gcgattaagt tgggtaacgc cagggttttc 300
   ccagtcacga cgttgtaaaa cgacggccaa agcttccaac ctgctgagaa gaacaactga 360
   cggtcataag gagagggagc ttttcgatag gtgccgtgca gttcaaagag ttagttagca 420
   gtaggatgaa gatttttgca catggcaatg agaagttaat tatggtgtag gcaacccaaa 480
   tgaaacacca aaatatgcac aagacagttt gttgtattct gtagtacaga ataaactaaa 540
   gtaatgaaag aagatggtgt tagaaaatga aacaatatta tgagtaatgt gtgagcatta 600
   tgggaccacg aaataaaaaa agaacatttt tatgagcagt gtgttctcaa tgagccttga 660
   atgttatcac ccaggataag aaacccttaa gcaatgaaac atgcaagcgt ttaatgtgca 720
   aagttggcat tctccacgac ataatgcaaa agaagatata atctatgaca tagcaagtca 780
   tgcatcattt catgcctctg tcaacctatt catttctagt catctaggta agtatcttaa 840
   gctaaagtgt tagaacttcc catacataag tcataactga tgacaattgg gtgtaacaca 900
   tgacaaacca gagagtcaag caagataaag caaaaggatg tgtacataaa actacagagc 960
   tatatgtcat gttgcgaaaa gaggagagct tataagacaa gccatgactc aaaaaaaatt 1020
   cacatgccta ctgtggccca tatatcatgc aacaatccaa aaactcacag gtctcggtgt 1080
   tgatcgtgtc aacatgtgac caccctaaaa actcttcact aaatattaaa gtattgctag 1140
   aacagagctt caagatataa gtcatgatca ccaacaacca tgttcaaaaa gaaatagaaa 1200
   gctatggcac agcaacaaaa agcaaaagca tgcatggata taatctttaa catcatccat 1260
   gtcatattgc aaaagaaaga aagagagaac aatacaaatg atgtgtcaat tacacatcca 1320
   tcattatcca tccaccttcc gtgtaccaca cttcatatat catgagtcac ttcatgtctg 1380
   gacattaaca aactctatct taacattcaa atgcatgaga ctttatctca ctataaatgc 1440
   acaatgattt agcattgttt ctcacaaaac cattcaagtt cattagtact acaacaacat 1500
   ggcatccata aatcgcccca tagttttctt cacagtttgc ttgttcctct tgtgcaatgg 1560
   ctctctagcc gaggacccgc agggcgacgc cgcccagaag accgacacca gccaccacga 1620
   ccaggaccac ccgaccttca acaagatcac cccgaacctc gccgagttcg ccttcagcct 1680
   ctaccgccag ctcgcccacc agagcaacag caccaacatc ttcttcagcc cggtgagcat 1740
   cgccaccgcc ttcgccatgc tcagcctcgg caccaaggcc gacacccacg acgagatcct 1800
   ggagggcctc aacttcaacc tcaccgagat cccggaggcc cagatccacg agggcttcca 1860
   ggagctcctc cgcaccctca accagccgga cagccagctc cagctcacca ccggcaacgg 1920
   cctcttcctc agcgagggcc tcaagctcgt ggacaagttc ctggaggacg tgaagaagct 1980
   ctaccacagc gaggccttca ccgtgaactt cggcgacacc gaggaggcca agaagcagat 2040
   caacgactac gtggagaagg gcacccaggg caagatcgtg gacctcgtga aggagctcga 2100
   ccgcgacacc gtgttcgccc tcgtgaacta catcttcttc aagggcaagt gggagcgccc 2160
   gttcgaggtg aaggacaccg aggaggagga cttccacgtg gaccaggtga ccaccgtgaa 2220
   ggtgccgatg atgaagcgcc tcggcatgtt caacatccag cactgcaaga agctcagcag 2280
   ctgggtgctc ctcatgaagt acctcggcaa cgccaccgcc atcttcttcc tcccggacga 2340
   gggcaagctc cagcacctgg agaacgagct cacccacgac atcatcacca agttcctgga 2400
   gaacgaggac cgccgcagcg ccagcctcca cctcccgaag ctcagcatca ccggcaccta 2460
   cgacctcaag agcgtgctcg gccagctcgg catcaccaag gtgttcagca acggcgccga 2520
   cctcagcggc gtgaccgagg aggccccgct caagctcagc aaggccgtgc acaaggccgt 2580
   gctcaccatc gacgagaagg gcaccgaggc cgccggcgcc atgttcctgg aggccatccc 2640
   ggtgagcatc ccgccggagg tcaagttcaa caagccgttc gtgttcctca tgatcgagca 2700
   gaacaccaag agcccgctct tcatgggcaa ggtcgtcaac ccgacccaga agtgagctcg 2760
   cgaccgcaaa ccgaagtcgg cggcttttct gctgcaaaaa cgctggactg gcatgaactt 2820
   cggtgaaaaa cc 2832
<210> 3
   <211> 20
   <212> DNA
   <213> Artificial Sequence
<220>
   <221> misc_feature
   <222> (1)..(20)
   <223> Forward primer for identifying the positive transformed plants
<400> 3
   gagggtgtgg aggctcttgt 20
<210> 4
   <211> 22
   <212> DNA
   <213> Artificial Sequence
<220>
   <221> misc_feature
   <222> (1)..(22)
   <223> Reverse primer for identifying the positive transformed plants
<400> 4
   gcccttgaag aagatgtagt tc 22
<210> 5
   <211> 22
   <212> DNA
   <213> Artificial Sequence
<220>
   <221> misc_feature
   <222> (1)..(22)
   <223> Forward primer for amplifying the AAT coding region
<400> 5
   gcatccataa atcgccccat ag 22
<210> 6
   <211> 22
   <212> DNA
   <213> Artificial Sequence
<220>
   <221> misc_feature
   <222> (1)..(22)
   <223> Reverse primer for amplifying the AAT coding region
<400> 6
   gcccttgaag aagatgtagt tc 22

## Description

### Field of the Invention

The present invention belongs to the field of genetic engineering, and particularly relates to a method for preparing, isolating and purifying OsrAAT from transgenic rice seeds.

### Background of the Invention

Human α 1- antitrypsin (AAT), also known as human α1- protease inhibitor (α1- PI), is serine-enriched protease inhibitor in human peripheral blood. It is mainly synthesized in the liver, and inhibits neutrophil elastase in the lung (Blank, Brantly, 1994). AAT deficiency is a hereditary disease associated with emphysema and liver disease (Eriksson, 1996). Deliveryof human plasma-derived α1-antitrypsin (plasma-derived AAT, pAAT) by intravenous injection is the only viable clinical treatment for patients with AAT deficiency (Heresi and Stoller, 2008). In addition, AAT has many therapeutic uses, for example, in the prevention of Type I diabetes in mice, the treatment of skin diseases (Lewis, Shapiro et al., 2005; Brown, 2006), and plays an important anti-inflammatory effect in the innate immune system (Xu, Dai et al., 2001).

At present, commercially available pAAT is majorly produced from human plasma, the output of which is limited by blood supply, meanwhile, human-derived pAAT has the risk of transmitting new or unknown pathogens, thus it appears to be very complex to ensure its safety (Kamaukhova, Ophir, et al., 2006). In addition, in order to meet the market demands, alternative approaches have been developed for the production of α1-antitrypsin (rTTA) with cost-effectiveness. In 1983, *Escherichia coli* were first used for the production of inactive rAAT (Bollen et al., 1983). Afterward, the rAAT expressed in *Escherichia coli* has reached 38mg/L (Kamaukhova et al., 2004). However, since *Escherichia coli* expression system lacks post transcriptional modification, the rAAT expressed therein is only used for laboratory studies. Yeast as an eukaryotic expression system can perform high-mannose-type glycan modifications (Cregg, Cereghino et al., 2000), but such modifications are different from human glycan structures. Deletion and abnormity of glycosylation are the main problems that interfere with the expression of recombinant glycoprotein in yeast expression system By fed-batch culture of yeast, the large-scale production of rAAT has reached a high yield of 1.23g/L (Tamer and Chisti, 2001), however, pharmacokinetic studies show that the rAAT produced by yeast is rapidly cleared from the blood (Casolaro, Fells et al., 1987). Also, some studies used *Aspergillusniger* to express rAAT because its glycosylation pattern is more similar to that of mammals (Maras, van Die et al., 1999; Gerngross 2004; Ward, Lin et al., 2004; Nevalainen, Te'o et al., 2005). However, the glycan modifications in the system are not studied, and the expression level of 50mg/L is still very low. Therefore the rAAT expressed in this system is also limited to laboratory studies.

Animal expression systems such as mice, rabbits, goats and sheep were also successfully used to express rAAT (Carlson, Rogers et al., 1989; Archibald, McClenaghan et al., 1990; Massoud, Bischoff et al., 1991; Wright, Carver et al., 1991; Carver, Wright et al., 1992; Ziomek, 1998). A large scale of rAAT were also produced from sheep milk (palrymple and Garner, 1998) and goat milk (Ziomek, 1998). The purity of rAAT isolated from transgenic sheep milk reached 99.9%, however, in human bodies, trace amounts of natural sheep AAT and α1-antichymotrypsin can induce a systemic antibody response (Spencer, Humphries et al., 2005). Plant expression systems were also used for the production of rAAT, including rice cell culture (Terashima, Murai et al., 1999), transgenic tomato (Agarwal, Singh et al., 2008) and chloroplasts (Nadai, Bally et al., 2009). Among them, the expression levels of rAAT in transgenic tomato and chloroplasts reached 1.55% and 2% of total soluble protein, respectively. The yield of rAAT in rice cell culture reached 200 mg/L. It is found recently that the endosperm cells of cerealcrops are very potential expression systems that can be used for the production of recombinant protein. Rice endosperm has been used to express various recombinant pharmaceutical proteins, such as human lactoferrin (Suzuki, Kelleher et al., 2003), human lysozyme (Yang, Guo et al., 2003), rhIGF-1 fusion and human granulocyte-macrophage colony-stimulating factor (Ning, Xie et al., 2008; Xie, Qiu et al., 2008). Recently, rice seeds are successfully applied to the large scale production of recombinant human serum albumin (He, Ning et al., 2011). These studies indicated that rice endosperm is a cost-effective and safe expression platform for drug proteins.

Though rAAT expression with different expression systems has made the progress, the large scale production of plant-derived recombinant human antitrypsin is still restricted by the expression level. In addition, so far it is still absent of the use of rice endosperm for large-scale production, isolation and purification of recombinant human antitrypsin from rice seeds.

### Summary of the Invention

One object of the invention is to provide a method for isolating and purifying OsrAAT from transgenic rice seeds containing OsrAAT, comprising the steps of:
(1) preparing OsrAAT extract from the transgenic rice seeds containing *OsrAAT* as raw material;
(2) subjecting the OsrAAT extract to anion exchange chromatography as a primary purification, to obtain primary OsrAAT elution fraction;
(3) subjecting the primary OsrAAT elution fraction to composite chromatography of cation exchange with metal chelation chromatography as a secondary purification, to obtain the secondary OsrAAT elution fraction;
(4) subjecting the secondary OsrAAT elution fraction to composite chromatography of anion exchange with hydrophobic chromatography as a final purification, to obtain purified OsrAAT.

Further, the method comprises the following steps of:
(1) using transgenic rice seeds containing *OsrAAT* as raw material, hulling the paddy rice into half polished rice and grinding it into milled rice with a fitness of 80-100 mesh; mixing the milled rice with an extraction buffer in a weight (kg)/volume (L) ratio of 1:5-1:10 and extracting for 1 hour at room temperature; subjecting the resultant mixture to pressure filtration with a filter-cloth-type plate-and-frame filter presser to obtain clear OsrAAT extract; wherein the components of the extraction buffer are: 20-25mM phosphate buffer, 1-4mM mercaptoethanol, pH6.9-7.1;
(2) performing the primary purification on a DEAE Sepharose FF chromatography column, equilibrating with 8-12 column volumes of pH 6.9-7.1, 20-25mM phosphate buffer with a flow rate of 100-180cm/h; using the OsrAAT extract of step 1as a loading sample, wherein the sample having a conductivity of 2-3.5ms/cm and a pH of 6.8-7.0; eluting the sample with pH 6.8-7.1, 100-110mM PB buffer at a flow rate of 100-180cm/h, and collecting the elution fraction containing OsrAAT, to obtain the primary OsrAAT elution fraction;
(3) performing the secondary purification on a cation exchange Macroprep CHT-I chromatography column with metal chelation capacity, equilibrating with 8-12 column volumes of pH 6.9-7.2, 5-12mM phosphate buffer with a flow rate of 100-150cm/h; diluting the primary OsrAAT elution fraction of step 2 up to four folds over the original volume as a loading sample, wherein the sample having a conductivity of 2-3.5ms/cm and a pH 6.8-7.0; eluting the sample with pH 6.8-7.1, 100-110mMPB buffer at a flow rate of 100-180cm/h, and collecting the elution fraction containing OsrAAT, to obtain the secondary OsrAAT elution fraction;
(4) performing the final purification on a chromatography column with cation exchange and hydrophobic characteristics, equilibrating with 8-12 column volumes of pH 7.5-8.2, 8-12mM phosphate buffer with a flow rate of 100-180cm/h; using the secondary OsrAAT elution fraction as a loading sample, wherein the sample having a conductivity of 2-3.5ms/cm and a pH 6.8-7.1; eluting the sample with pH 6.6-7.0, 46mM PB, 400mM NaCl buffer at a flow rate of 100-180cm/h, and collecting the elution fraction containing OsrAAT to obtain purified OsrAAT.

Further, the method may comprise the following steps of:
(1) using transgenic rice seeds containing *OsrAAT* as raw material, hulling the paddy rice into half polished rice and grinding it into milled rice with a fitness of 80-100 mesh; mixing the milled rice with an extraction buffer in a weight/volume ratio of 1kg :10L and extracting for 1 hour at room temperature; subjecting the resultant mixture to pressure filtration with a filter-cloth-type plate-and-frame filter press to obtain clear OsrAAT extract; wherein the components of the extraction buffer are: 20mM phosphate buffer, 1mM mercaptoethanol, pH 7.0;
(2) packing an Econo-column 15/20 chromatography column with 20ml of DEAE Sepharose FF, equilibrating the column with 200ml of pH 7.0, 20mM phosphate buffer with a flow rate of 150cm/h; using the OsrAAT extract as a loading sample, wherein the sample having a conductivity of 2.6ms/cm and a pH 6.95; eluting the sample with pH 7.0, 108mM PB buffer at a flow rate of 150cm/h, and collecting the elution fraction containing OsrAAT, to obtain the primary OsrAAT elution fraction;
(3) packing an Econo-column 15/20 chromatography column with 20ml of Macroprep CHT-I, equilibrating the column with 200ml of pH 7.0, 20mM phosphate buffer at a flow rate of 150cm/h; diluting the primary OsrAAT elution fraction of step 2 to four folds its original volume as a loading sample, wherein the sample having a conductivity of 3.0ms/cm and a pH 6.9; eluting the sample with pH 7.0, 108mM PB buffer at a flow rate of 150cm/h, and collecting the elution fraction containing OsrAAT, to obtain the secondary OsrAAT elution fraction;
(4) packing an Econo-column 15/20 chromatography column with 10ml of Capto Adhere, equilibrating the column with 200ml of pH 8.0, 10mM phosphate buffer at a flow rate of 150cm/h; using the secondary *OsrAAT* elution fraction as a loading sample, wherein the sample having a conductivity of 3.0 ms/cm and a pH of 6.9; eluting the sample with pH 6.8, 46mM PB, 400mM NaCl buffer at a flow rate of 150cm/h, and collecting the elution fraction containing OsrAAT, to obtain purified OsrAAT.

### Description of Drawings

Figure 1 is a schematic diagram of the structure of the plasmid pOsPMP02.
Figure 2 is a schematic diagram of the structure of the plasmid pOsPMP 131.
Figure 3 is a schematic diagram of the structure of the plasmid pOsPMP132.
Figure 4 is a schematic diagram of the structure of the plasmid pOsPMP135.
Figure 5 is the result of Western hybridization, showing that the expressed OsrAAT was obtained in the endosperm cells in 9 different strains of transgenic rice.
Figure 6 is the result of Southern hybridization, wherein enzyme digestion was performed with *EcoRI*, *HindIII*, and both *EcoRI* and *HindIII*, respectively.
Figure 7 shows the expression levels of OsrAAT in different plants.
Figure 8 is an electrophorogram of anion exchange chromatography performed on different chromatography resinsas primary purification; wherein, Fig. 8A: DEAE Sepharose FF resin; Fig.8B: Macroprep DEAE resin; Fig. 8C: Capto Q resin; M: molecular marker, S: loaded sample, FT: flow-through peak, Elu: OsrAAT elution peak, Elu1: impurity elution peak, Elu2: OsrAAT elution peak, CIP: cleaning in place.
Figure 9 is an electrophorogram of composite chromatography performed on Macroprep CHT-I as primary purification; wherein, M: molecular marker, S: loaded sample, FT: flow-through peak, Elu: OsrAAT elution peak, CIP: cleaning in place.
Figure 10 is an electrophorogram of hydrophobic chromatography performed on different chromatography resins as secondary purification; wherein, Fig. 10A: Phenyl Sepharose HP resin, Fig. 10B: Phenyl sepharose FF HS resin, Fig. 10C: Octylsepharose FF resin, M: molecular marker, S: loaded sample, FT: flow-through peak, Elu: OsrAAT elution peak, CIP: cleaning in place.
Figure 11 is an electrophorogram of composite chromatography performed on different chromatography resins as secondary purification; wherein, Fig. 11A: Macroprep CHT-I resin, Fig. 11B: Capto MMC resin, Fig. 11C: Capto Adhere; M: molecular marker, S: loaded sample, FT: flow-through peak, Elu: OsrAAT elution peak, Elu1: impurity elution peak, Elu2: OsrAAT elution peak, CIP: cleaning in place.
Figure 12 is an electrophorogram of composite chromatography performed on Capto Adhereas final purification; wherein, M: molecular marker, S: loaded sample, FT: flow-through peak, Elu: OsrAAT elution peak, CIP: cleaning in place.
Figure 13 is an electrophorogram of affinity chromatography performed on different chromatography resins as final purification; wherein, Fig. 13A: AAT-select resin, Fig. 13B: ConA sepharose 6B resin, M: molecular marker, S: loaded sample, FT: flow-through peak, Elu: OsrAAT elution peak.
Figure 14 is an electrophorogram of crude rAAT-containing extract that was purified sequentially by anion exchange chromatography, cation exchange with metal chelation chromatography, anion exchange with hydrophobic chromatography; wherein from left to right, the resins are DEAE sepharose FF, Macroprep CHT-I and Capto Adhere; M: molecular marker, S: loaded sample, FT: flow-through peak, Elu: OsrAAT elution peak.
Figure 15 is an HPLC chromatogram of the purified OsrAAT (HPLC-SEC).
Figure 16 shows the analysis results of the biological activity of OsrAAT; wherein, on the left are SDS-PAGE analysis results of band shift; on the right are the results of Western hybridization; M: molecular marker, 1: OsrAAT from rice 132-17 T1 generation plants, 2: human plasma AAT, 3: OsrAAT added with porcine elastase, 4: human plasma AAT added with porcine elastase, 5: extract of rice variety Zhonghua 11.
Figure 17 shows the determination results of porcine elastase inhibitory activity of OsrAAT.

### Detailed Description of the Invention

The characteristics and advantages of the present invention will be described in detail in conjunction with the accompanying drawings. The examples are only provided to illustrate the present invention, but not intended to limit the other content disclosed by the invention in any way.

In the following examples, Macroprep CHT-I was available from BIO-RAD company; DEAE Fast Flow, Macroprep-DEAE, Capto Q, Phenyl sepharose HP, Phenyl sepahrose FF HS, Octyl sepharose FF, Capto MMC, Capto Adhere, AAT-select, an dConA Sepharose 6B were available from GE Healthcare company; Econo-column 15/20 chromatographic column was purchased from BIO-RAD company; XK16/20 chromatographic column was purchased from GE Healthcare company. Unless otherwise specified, other materials and reagents were ordinary commercially available.

### [Example 1] Construction of Recombinant Human Antitrypsin Vector Specifically Expressed in Rice and Preparation of Transgenic Rice Plant.

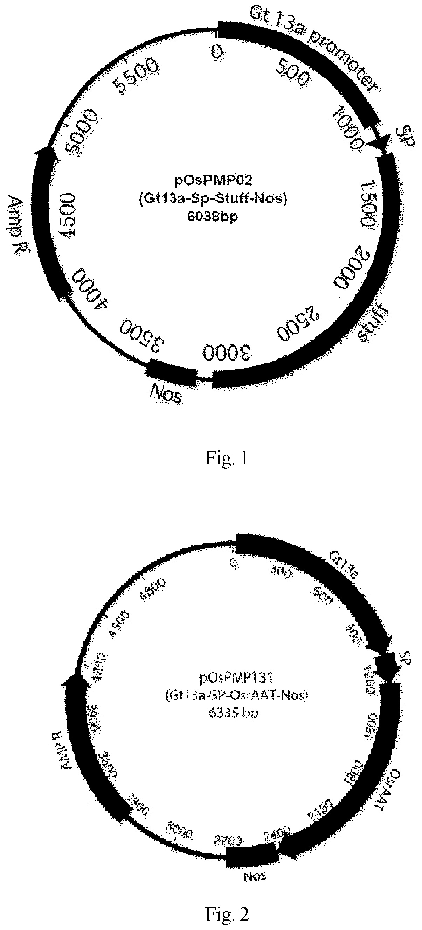

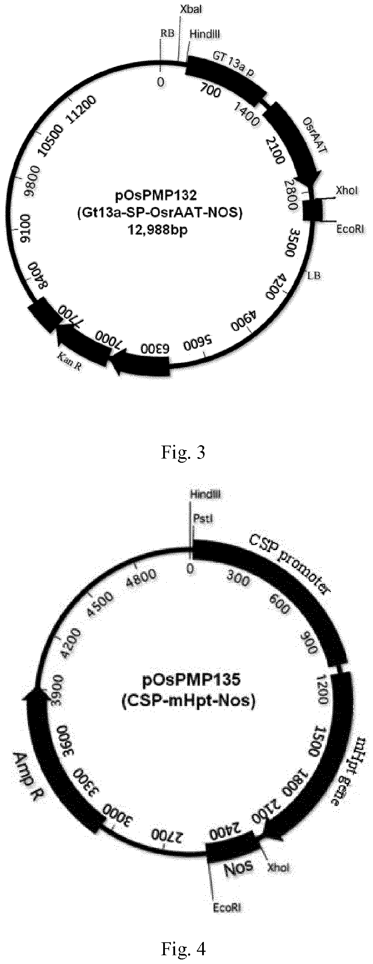

Human *AAT* genes (GenBank accession number: M001002235) were synthesized by Heron Blue Biotech Corporation according to rice preferred genetic codons. 46.5% of human α1-antitrypsin (AAT) genetic codons were optimized and 18.1% of human α1-antitrypsin nucleotides were altered, particularly as shown in SEQ ID NO.1, but the corresponding amino acid sequence was not changed. The present disclosure employed rice specific promoter *Gtl3a* and its signal peptide to express recombinant human antitrypsin gene in rice endosperm cells, particularly the recombinant human antitrypsin vector specifically expressed in rice of the present disclosure was constructed and the transgenic rice plants were screened according to the method of patent publication number CN100540667, wherein the recombinant human serum albumin thereof was replaced with recombinant human antitrypsin of the present disclosure. The plasmid pOsPMP02 as shown in Figure 1 was used to construct rice endosperm-specific expression cassette. The synthesized codon-optimized human AAT gene (SEQ ID NO.1) was digested with *MylI* and *XhoI* and cloned into pOsPMP02, the resulting construct was designated as pOsPMP131, as shown in Figure 2; and then pOsPMP131 was digested with *HindIII* and *EcoRI,* the 2832bp fragment containing *Gtl3a* promoter and signal peptide, the codon-optimized ATT gene and Nos Terminator (as shown in SEQ ID NO.2) was ligated into a binary vector JH2600, an *Agrobacterium-mediated* plasmid, the resulting construct was designated as pOsPMP132, as shown in Figure 3. The plasmid pOsPMP132 and pOsPMP135 plasmid as shown in Figure 4 were transformed into *Agrobacterium tumefaciens* strain EHA105, respectively (Invitrogen company, USA). pOsPMP132 and pOsPMP135 were co-transformed into the callus derived from a rice variety, Zhonghua 11via *Agrobacterium tumefaciens*-mediated transformation. And then they were cultured, screened and induced to generate plantlets. The positive transformed plants with resistance of hygromycin were then identified by PCR amplification with the forward primer starting from signal peptide being (5'-GAGGGTGTGGAGGCTCTTGT-3') and the reverse primer sequence starting from the AAT gene being (5' GCCCTTGAAGAAGATGTAGTTC 3'). Total of 23 independent transgenic rice plants containing recombinant human α 1-antitrypsin and 12 independent transgenic rice plants containing high-yield recombinant human α1-antitrypsin were obtained.

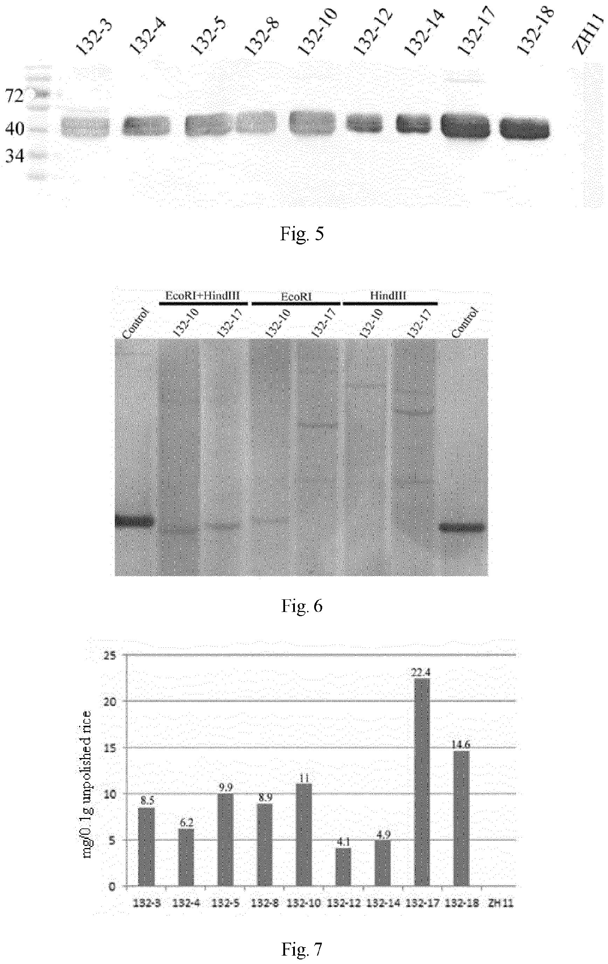

Further, Western blotting was used to detect whether OsrAAT expressed in transgenic rice grain was harvested from the endosperm cells. About 200mg of rice seeds was ground with 600µl PBS at 4°C, and then centrifuged at 10,620 xg for 5 minutes, to obtain 40 ml of crude protein extract. The crude protein extracts and 200ng of human blood-derived AAT were subjected to 12% SDS-PAGE gel and finally stained with 0.1% of Coomassie Brilliant blue R-250. As shown in Figure 5, nine transgenic rice lines were identified to express *OsrAAT* in rice endosperm In addition, Southern blotting was used to identify the T1 plants of above two transgenic rice strains, 132-17 and 132-10. For detail, about 100mg of leaves were respectively ground in liquid nitrogen and extracted with quick type plant genome DNA extraction system (Tiangen Biotech Co., LTD, China) to obtain genomic DNA. The genomic DNA was digested with *EcoRI*, *HindIII*, as well as *EcoRI*/*HindIII* (New England Biolabs) at 37°C for 8 hours, respectively. And then they were separated by 0.8% agarose gel and blotted onto MILLIPORE NY⁺ membranes. The procedures were described as the instructions of DIG High Prime DNA Labeling and Detection Starter Kit I (Roche). The 645 bp probe containing the coding region of AAT was amplified using primers (5'-GCATCCATAAATCGCCCCATAG -3' and 5'- GCCCTTGAAGAAGATGTAGTTC -3'), which was used for the hybridization. The results showed that the two fragments could be detected after digestion with *EcoRI* or *HindIII*, which was consistent to the results of genetic analysis. The results from the double digestion with *EcoRI*/*HindIII* indicated that the insertion contain entire expression cassette, which was the same as the expression vector digested with the same enzymes as shown in Figure 6.

In this example, the expression levels of OsrAAT in nine transgenic rice lines were measured by porcine elastase inhibitory activity assay. The results showed that the expression level of OsrAAT reached 0.4-2.24mg/per gram brown rice as shown in Figure 7. A highest line 132-17 with expression level of OsrAAT was chosen forward to next generation for further studies. To characterize the transgenic line, 132-17, the genetic segregation of its T1 seeds was analyzed. The results showed that 51 seeds expressed *OsrAAT*, 5 seeds did not expressed OsrAAT, fitting with two locimodel (15:1, CHITEST *p*=0.408). Finally, the transgenic rice lines with high expression level of *OsrAAT* were obtained.

### [Example 2] Optimization the Chromatography Resins and Elution Conditions for Isolation and Purification of OsrAAT from Transgenic Grains

Optimized chromatography conditions to isolate and purify OsrAAT from rice seeds in the present disclosure are as follows:

### 1. Primary Purification of OsrAAT

### 1.1 Optimization of Anion Chromatography Resins and Elution Conditions in Primary Purification Step

The present inventors defined the desirable chromatography resin as the anion resins with high flow rate, including such as Macro-prep DEAE (from BIO-RAD company), DEAE Sepharose FF and Capto Q (from GE company).

It was found that Macro-prep DEAE, DEAE Sepharose FF and Capto Q can be used for the purification of OsrAAT. However, the best purification efficiency of OsrAAT was obtained from both DEAE SepharoseFF and Macro-prep DEAE, while Capto Q was better under low salt condition. However, under the same loading conditions, the two weak anion resins showed a longer retention time and better separation efficiency than Capto Q. Due to the high content of pigments and polysaccharides in the rice seeds, they could bind to the anion resins, resulting in a decrease in loading capacity and purity. It was more obvious on Capto Q resin. With increase of the salt concentration to remove the impurities during the equilibrium procedures, DEAE Sepharose FF showed obvious tolerance to higher salt concentration, which could effectively separate the target protein and host proteins. However, the target protein flowed out when the Macro-prep DEAE was used and when the salt concentration was over10mM PB. Our results indicated that DEAE Sepharose FF had higher flow rate than Macro-prep DEAE under the condition of equivalent purification ability because DEAE Sepharose FF has an average particle size of 75 µm, while Macro-prep DEAE has an average particle size of 50 µm.

OsrAAT-containing extract with pH7.0 and low conductivity condition was loaded into a chromatography column packed with DEAE Sepharose FF to ensure that OsrAAT can fully bind on the column. Since the target protein had serious activity loss at low pH, PB gradient elution method was chosen instead of pH gradient elution. The results indicated that the target protein was mainly eluted between 10-20% 500mMPB, but no impurity was eluted under the condition of less than 10% 500mM PB. This means that it was not suitable to need removal step of impurity before the target protein was eluted. Accordingly, it was believed that under this pH condition, it was difficult to improve the purity of the target protein by increasing the salt concentration inelution solution only. Regarding to purity and recovery rate of target protein, 108mMPB, pH7.0 are optimized elution conditions.

### 1.2 Selection of Composite Chromatography Resin for Primary Purification

Regarding to the stability of the target protein, the purification procedures can only be carried out under neutral pH condition in which conventional cation exchange resin will inevitably cause the target protein not to be absorbed on the column, but the cation exchange resins have advantages for removing impurities such as pigments. The present inventor, therefore, selected Macroprep CHT-I (cation exchange with metal chelation capacity) for primary purification.

The samples interacted with the resin through the negatively charged phosphate and positively charged calcium ions of Macroprep CHT-I. The results showed that Macroprep CHT-I had effectively enriched the target protein, but its flow rate and loading capacity were slightly less than that of DEAE Sepharose FF. Unfortunately, the pigments in the extracts were tightly bound on the resin, though the extract sample was loaded on the resinonly once, so that about 5% column volume of resin cannot be regenerated. Therefore, it is not suitable to use the resin for primary purification though Macroprep CHT-I has better purification effect.

### 1.3 Determination of Chromatography Resin and Elution Conditions for Primary Purification

Regarding to various factors, DEAE Sepharose FF is the preferred chromatography resin and 108mM PB and pH7.0 are optimized elution conditions for primary purification.

### 2. Secondary Purification of OsrAAT

### 2.1 Selection of Chromatography Hydrophobic Resin and Optimization of Elution Conditions for Secondary Purification

Hydrophobic resins have a better capacity to remove the non-specific impurities in transgenic rice. Various hydrophobic resins with similar properties were tested for this purification step, respectively, including Phenyl Sepharose HP, Phenyl Sepharose FF (HS) and Octyl Sepharose FF. The differences between Phenyl Sepharose FF (HS) and Phenyl Sepharose HP are spherical substrate diameters and ligand densities. The average particle size of the former is about 3 times larger than that of the latter. Thus, Phenyl Sepharose FF has higher working flow rate though it brings inconvenience to the application, while Phenyl Sepharose HP has fine particle size, and it has higher resolution and can achieve better purification effect.When the salt concentration of the sample was adjusted to reach a final concentration of ammonium sulfateof 0.75M, 1.2M, 1.5M, and loaded on a chromatography column packed with Phenyl Sepharose HP as flow-through fraction, and then 50% water-eluted fraction and pure water-eluted fraction were collected. Each fraction was detected by electrophoresis. The results showed that the good removal effect of impurities was obtained under each concentration of ammonium sulfate, the purity of target protein of the flow-through fraction reached 70%. However, the target protein retained on the column was increased with the increase of the concentration of ammonium sulfate, which seriously not only affect the recovery of target protein, but also reduce the biological activity of rAAT in the flow-through sample.

We found that the hydrophobicity of Phenyl Sepharose FF was higher than that of Phenyl Sepharose HP. Under the condition of 0.8M ammonium sulfate, 80% of the target protein was retained on the column. Flow-through fraction, 50% water-eluted fraction and pure water-eluted fraction were collected, which were detected by electrophoresis. The results showed that the purity of the target protein was significantly improved, the protein purity of 40% water-eluted fraction reached 80%, but the biological activity was still seriously lost.

Compared with the above resins, Octyl Sepharose FF has the same matrix, but different ligands. So, its hydrophobicity is weaker. Since the rAAT expressed in the transgenic rice has different degrees of glycosylation modification, the difference of various rAAT can make them to be selectively separated on Octyl resin. When the concentration of ammonium sulfate of the sample was adjusted up to 1M and then loaded to Octyl SepharoseFF chromatography column, flow-through fraction, 40% water-eluted fraction and pure water-eluted fraction were collected and then detected by electrophoresis. The results showed that the purity of the target protein was significantly increased, and the loss of the biological activitywas distinctly decreased compared to Phenyl sepharose FF(HS) and Phenyl Sepharose HP, but the activity recovery was still not high enough.

In summary, the three hydrophobic resins have high capacity to purify OsrAAT, but those are not suitable for the purification of OsrAAT due to their influence on the activity of the target protein. When the sample passes through the hydrophobic resin, the hydrophobic environment may change the tertiary structure of target protein to cause biological activity loss.

### 2.2 Selection of Composite Chromatographic Resin and Elution Conditions for Chromatography for Secondary Purification

The present inventors defined the composite resins with high flow rate as the desirable chromatography resin, including such as Macroprep CHT-L, Capto MMC and Capto Adhere.

Capto MMC is a chromatography resin with cation exchange and hydrophobic characteristics. Two salt concentrations of the samples with low salt of 100 mM PB and high salt of 1.5 M ammonium sulfate were tested. It was found that some of the target protein being strongly hydrophobic still retained in the resin when the sample contained low salt concentration. The purity of the target protein was relatively low in both the flow-through sample and the elution fraction samples. High purity of the target protein was obtained when high salt loading condition was used. The protein purity from flow-through fraction reached up to 85%, but the biological activity was still low.

It was found that the best purification effect and the highest activity recovery were obtained using Macroprep CHT-I. Although Capto MMC under high salt condition had relatively better purification effect, the biological activity was not good enough. Capto Adhere had poor purification effect, but high activity recovery, wherein both Macroprep CHT-I and Capto Adhere can be used for the purification of recombinant human antitrypsin. Macroprep CHT-I can remove more impurity proteins, while Capto Adhere can specially remove some impurity proteins that cannot be removed by Macroprep CHT-I. Macroprep CHT-I resin also has advantages of ease of packing on the column due to its rigid matrix, excellent stability under high concentrations of sodium hydroxide, simple cleaning process and cost-effectiveness over Capto Adhere. Taking various factors into consideration, Macroprep CHT-I is a preferable composite chromatography resin. Capto Adhere could be a suitable chromatography resin for final purification.

Based on features of CHT resin itself is required pH not less than 7.0 and phosphate sensitivity, we employed the basic elution conditions of 108 mM PB, pH 7.0 according to phosphate gradient elution study. The elution conditions were further optimized. The conditions of Macroprep CHT-I to completely remove impurities were employed according to sodium chloride gradient elution method, and compared with the original elution conditions. The results showed that the purity was distinctly improved with an HPLC purity being up to 85%, but the recovery was reduced nearly 10% after adding a washing step for removal of the impurities. Those impurities were expected to be removed by the subsequent purification step. Taking purification effect and recovery into consideration, optimized elution conditions are 108 mM PB, pH 7.0 in Macroprep CHT-I resin, without the step of washing impurities.

### 2.3 Determination of Chromatography Resin and Elution Conditions for Secondary Purification

Both hydrophobic resins and the composite resins with hydrophobic feature have obvious purification effect, but all of them have certain lost of biological activity, except for Capto Adhere with anion exchange and hydrophobicity. Capto Adhere as chromatographic resin for secondary purification does not affect the biological activity and exhibits a very good removal capacity of some impurity, but the removal capacity of the most impurityis not good enough. Macroprep CHT-I resin has significant advantages on removal capacity of most impurities and activity recovery. Thus, Macroprep CHT-I resin is the best choice of chromatography resin and 108 mM PB, pH 7.0 are optimized elution conditions for secondary purification.

### 3. Final Purification of OsrAAT

### 3.1 Selection of Chromatography Resin and Optimization of Elution Conditions for Final Purification

In previous study, Capto Adhere exhibited very good capacity to remove high molecular weight impurities that cannot be removed by Macroprep CHT-I resin during secondary purification. Therefore, it was determined as preferred resin for final purification step.

OsrAAT-containing extract with pH 7.0 and a conductivity of 3.0 ms/cm was loaded on a chromatography column packed with CaptoAdhere. NaCl gradient elution and pH gradient elution protocol, respectively were used to study the primary elution conditions. The results showed that OsrAAT was gradually eluted with the increase of pH and the decrease of conductivity. When elution pH was about 6.8 and a conductivity was about 40ms/cm, approximately 80% target protein was eluted. Taking purification efficiency and recovery rate into consideration, 0.4M sodium chloride containing 46mMPB and pH 6.8 are the preferred elution conditions for final purification step.

### 3.2 Selection of Chromatography Affinity Resin and Elution Conditions for Final Purification

The present inventors defined desirable resins as affinity resins with high working flow rate, including such as ConA Sepharose 6B and AAT-select. The experiment results indicated that both ConA Sepharose 6B and AAT-select had a good purification effect, and can be used for the purification of recombinant human antitrypsin. Recombinant target protein is a protein collections with different degrees of glycosylation. ConASepharose 6B can separate the glycosylated OsrAAT and non-glycosylated OsrAAT. The biological activity assay indicated that OsrAAT activity was not dependent on the degree of glycosylation. High purity of OsrAAT can be obtained from elution fraction, meanwhile, non-glycosylated OsrAAT with activity flows through and was lost. So, the protein amount and activity recovery was not high. There are several advantages of AAT-select as an affinity chromatography resin specially for AAT purification. It can recovery all OsrAAT either glycosylated or non-glycosylated OsrAAT. Thus, all OsrAAT with different degrees of glycosylation modification can be captured and effectively separated from impurities. Furthermore, the cleaning process and cyclelife were superior to that of ConA Sepharose 6B. Take together, AAT-select is the preferred affinity chromatography resin.

### 3.3 Determination of Chromatography Resin and Elution Conditions of Final Purification

Both Capto Adhere and AAT-select can be used for OsrAAT purification. However, Capto Adhere can remove OsrAAT aggregates while AAT-select cannot. Its purity reached up to97% determined by HPLC assay, which was much higher than 85% of AAT-select. Target protein eluted from AAT-selected required 2M MgCl₂ in elution buffer, which increased the cost. Furthermore, it was difficult to handle the resulting eluent with high salt concentration. CaptoAdhere is easier to operate than that of AAT-select, including cleaning process and cost-effective.

Taken together, CaptoAdhere is the preferred resin for final purification, optimized elution conditions are 46 mM PB, pH 6.8, 0.4M sodium chloride.

### [Example 3] Isolation and Purification of OsrAAT

This example integrated the three purification steps, including the preferred resin and optimized elution conditions for each chromatography for isolation and purification of OsrAAT, which are described in Example 2.

### 1. Preparation of OsrAAT Sample for Chromatography

The paddy rice of transgenic rice line No. 132-17 was hulled to obtain half-polished grains and then ground with a fineness of 80-100 mesh. The milled rice was mixed with the extraction buffer (20 mM phosphate buffer, pH 7.0, 1mM mercaptoethanol) in a ratio of 1:10 (weight/volume, kg/L) and extracted for 1 hour at room temperature. The resultant mixture was subjected to pressure filtration to obtain clear OsrAAT extract for future chromatography using a filter-cloth-type plate-frame filter presser.

### 2. Primary Purification

### 2.1 Primary Purification by Anion Exchange Chromatography

### 2.1.1 Anion Exchange Chromatography Practiced by DEAE Sepharose Fast Flow

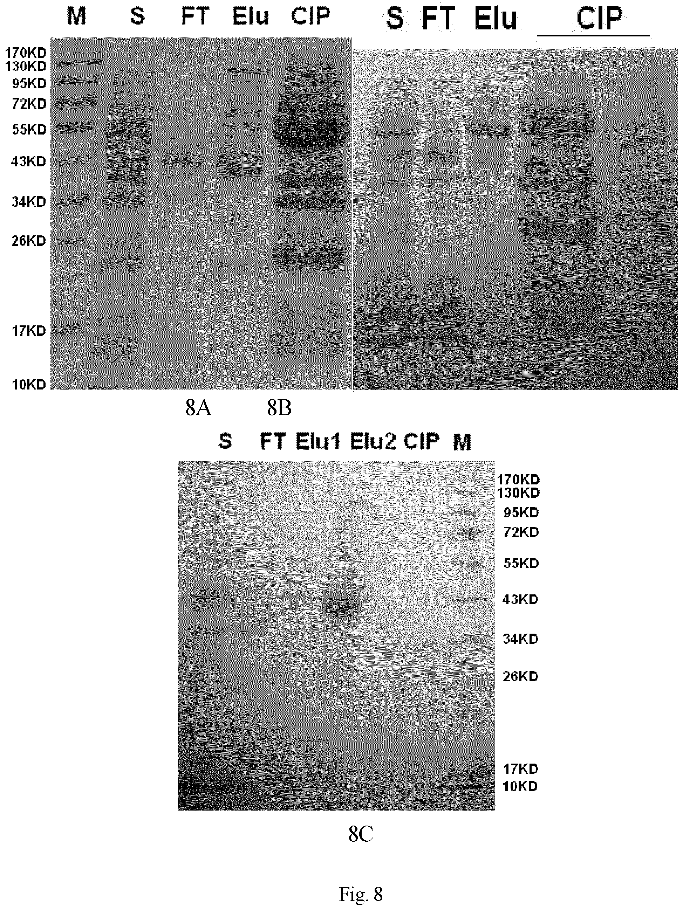

About 20 ml of DEAE Sepharose Fast Flow resin was packed on the Econo-column 15/20 chromatography column. It was equilibrated with 200 ml of equilibration buffer (20mM phosphate buffer; pH 7.0) at a flow rate of 150 cm/h until the pH value and the conductivity were constant to baseline. The sample with the conductivity of 2.6ms/cm and pH of 6.95 was loaded. The sample was eluted with the elution buffer (108mMPB, pH6.8) at a flow rate of 150cm/h. The OsrAAT-containing fraction was collected and β-mercaptoethanol was added to reach a final concentration of 4mM. The chromatography results are shown in Figure 8A.

### 2.1.2 Anion Exchange Chromatography Practiced by Macroprep-DEAE

About 16 ml of Macroprep-DEAE resin was packed onto the XK16/20 chromatography column. It was equilibrated with 200 ml of equilibration buffer (20mM phosphate buffer, pH 7.0) at a flow rate of 150 cm/h until the pH value and the conductivity were constant to baseline. The sample with the conductivity of 5.3ms/cm and the pH of 6.95 was loaded. The sample was eluted with the elution buffer (108mMPB, pH7.0) at a flow rate of 150cm/h. The flow-through fraction and elution fraction were collected. The chromatography results are shown in Figure 8B.

### 2.1.3 Anion Exchange Chromatography Practiced by Capto Q

About 15ml of Capto Q resin was packed onto the XK16/20 chromatography column. It was equilibrated with 200 ml of equilibration buffer (20mM phosphate buffer; pH 7.0) at a flow rate of 150 cm/h until the pH value and the conductivity were constant to baseline. The sample with the conductivity of 5.3ms/cm and the pH of 6.95 was loaded. The sample was eluted with the elution buffer (108mMPB, pH7.0) at a flow rate of 150cm/h. OsrAAT-containing fraction was collected and β-mercaptoethanol was added to reach a final concentration of 4mM. The chromatography results are shown in Figure 8C.

### 2.2 Primary Purification by the Composite Chromatography resins

Anion Exchange with Metal Chelation Chromatography Practiced by Macroprep CHT-I resin

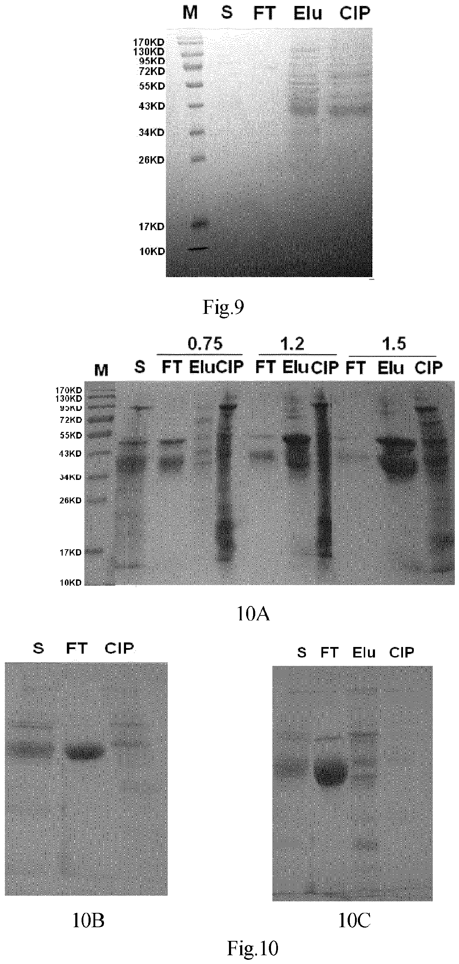

About 20 ml of Macroprep CHT-I resin was packed onto the Econo-column 15/20 chromatography column. It was equilibrated with 200ml of equilibration buffer (10mM phosphate buffer, pH7.0) at a flow rate of 150cm/h until the pH value and the conductivity were constant to baseline. The sample with the conductivity of 1.5ms/cm and the pH of 6.9 was loaded. The sample was eluted with the elutionbuffer (108mMPB, pH7.0) at a flow rate of 150cm/h. OsrAAT-containing fraction was collected and β-mercaptoethanol was added to reach a final concentration of 4mM.The chromatography results are shown in Figure 9.

The chromatography effect of exchange chromatography performed on the above four different resins are shown in Figures 8 and 9. OsrAAT-containing fraction eluted from DEAE Sepharosse Fast Flow exhibited the best purification effect and then was used for subsequent secondary purification.

### 3. Secondary purification

### 3.1 Secondary Purification using Hydrophobic Chromatography resins

### 3.1.1 Hydrophobic Chromatography Practiced by Phenyl Sepharose HP resin

About 20 ml of Phenyl Sepharose HP was loaded onto the XK16/20 chromatography column. It was equilibrated with 200ml of equilibration buffer (108mM phosphate buffer (pH 7.0) with 0.75 M, 1.2 M, 1.5 M ammonium sulfate, respectively;) at a flow rate of 150 cm/h until the pH value and the conductivity were constant baseline. OsrAAT-containing fraction from 2.1.1 (DEAE SepharoseFast Flow) was adjusted with ammonium sulfate until reaching a concentration of 0.75M, 1M and 1.5M, showing the conductivity be 95, 135, 165.0ms/cm, respectively. The pH was adjusted to pH 6.9. The samples were then loaded onto the column at the flow rate of 150cm/h. The flow-through fraction was collected and β-mercaptoethanol was added to reach a final concentration of 4mM.The results are shown in Figure 10A.

### 3.1.2 Hydrophobic Chromatography Practiced by Phenyl Sepahrose FF HS resin

About 20ml of Phenyl Sepahrose FF HS resin was packed onto the Econo-column 15/20 chromatography column. It was equilibrated with 200 ml of equilibration buffer (108mM phosphate buffer, 1.0M ammonium sulfate, pH 7.0) at a flow rate of 150 cm/h until the pH value and the conductivity were constant to baseline. OsrAAT-containing fraction from the 2.1.1 (DEAE Sepharose Fast Flow was adjusted with ammonium sulfate until reaching a concentration of 1M, making the conductivity be 135ms/cm and pH 6.9. The sample was then loaded onto the column at the flow rate of 150 cm/h. The flow-through fraction was collected and β-mercaptoethanol was added to reach a final concentration of 4mM. The results are shown in Figure 10B.

### 3.1.3 Hydrophobic Chromatography practiced by Octyl Sepharose FF resin

About 20 ml of OctylSepharose FF resin was packed onto the XK16/20 chromatography column. It was equilibrated with 200ml of equilibration buffer (20mM phosphate buffer; 1.0M ammonium sulfate; pH 7.0) at a flow rate of 150 cm/h until the pH value and the conductivity were constant to baseline. OsrAAT-containing fraction from 2.1.1 (DEAE Sepharose Fast Flow) was added with ammonium sulfate until reaching a concentration of 1M, making the conductivity to be 120.0ms/cm, the adjusted pH to be 6.9. The sample was then loaded on the column at a flow rate of 150 cm/h. The flow-through fraction was collected and β-mercaptoethanol was added to reach a final concentration of 4mM. The results are shown in Figure 10C.

### 3.2 Secondary Purification by the Composite Chromatography resins

### 3.2.1 Anion Exchange with Metal Chelation Affinity Chromatography Practiced by Macroprep CHT-I

20ml of the OsrAAT-containing fraction from 2.1.1 (DEAE Sepharose Fast Flow) was diluted to four times its original volume using pure water for use.

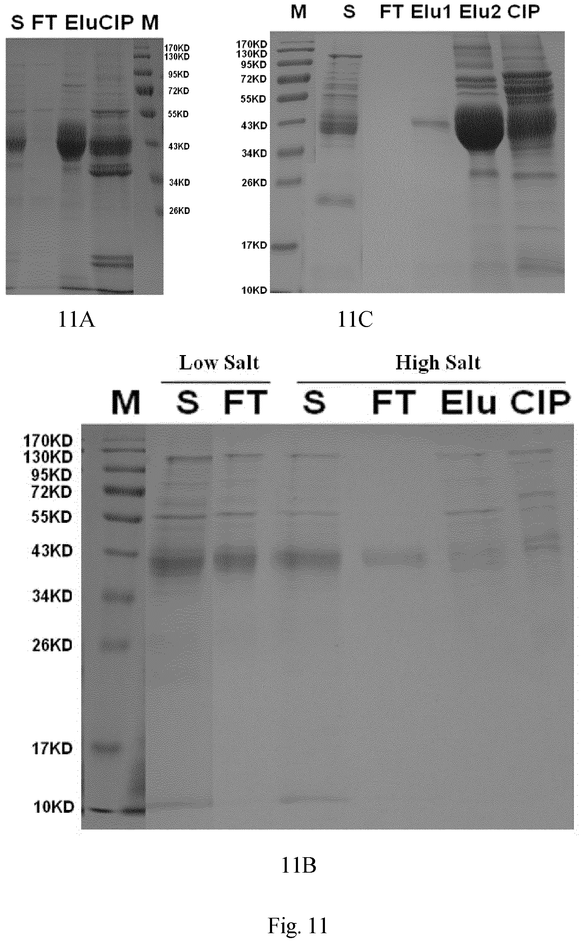

About 20 ml of Macroprep CHT-I resin was packed onto the XK16/20 chromatography column. It was equilibrated with 200ml of equilibration buffer (10mM phosphate buffer; pH 7.0) at a flow rate of 150cm/h until the pH value and the conductivity were constant to baseline. The sample with the conductivity of 3.0ms/cm and the pH of 6.9 was loaded. The sample was eluted with the elution buffer (108mMPB, pH7.0) at a flow rate of 150cm/h. OsrAAT-containing fraction was collected and β-mercaptoethanol was added to reach a final concentration of 4mM. The results are shown in Figure 11A.

### 3.2.2 Anion Exchange with Hydrophobic Chromatography Practiced by Capto MMC resin

About 20 ml of Capto MMC resin was packed onto the XK16/20 chromatography column. It was equilibrated with 200ml of equilibration buffer (20mM phosphate bufferpH 7.0) at a flow rate of 150 cm/h until the pH value and the conductivity were constant to baseline. The sample with the conductivity of 3.0ms/cm and the pH 6.9 was loaded.The sample was eluted with the elution buffer (108mMPB, pH7.0) at a flow rate of 150cm/h. The flow-through fraction and elution fraction were collected. The results are shown in Figure 11B.

### 3.2.3 Cation Exchange with Hydrophobic Chromatography Practiced by Capto Adhere

About 20 ml of Capto Adhere resin was packed onto the Econo-column 15/20 chromatography column. It was equilibrated with 200ml of equilibration buffer (10mM phosphate buffer, pH 8.0) at a flow rate of 150 cm/h until the pH value and the conductivity were constant to baseline. The sample with the conductivity of 3.0ms/cm and the pH of 6.9 was loaded. The sample was eluted with the elution buffer (46mMPB, 400mMNaCl, pH6.8) at a flow rate of 150cm/h. OsrAAT-containing fraction was collected and β-mercaptoethanol was added to reach a final concentration of 4mM. The results are shown in Figure 11C.

Based on the previous result, OsrAAT-containing fraction from Macroprep CHT-I was used for the final purification.

### 4. Final Purification

### 4.1 Final Purification by Composite Chromatography resins

### 4.1.1 Cation Exchange with Hydrophobic Chromatography Practiced by Capto Adhere

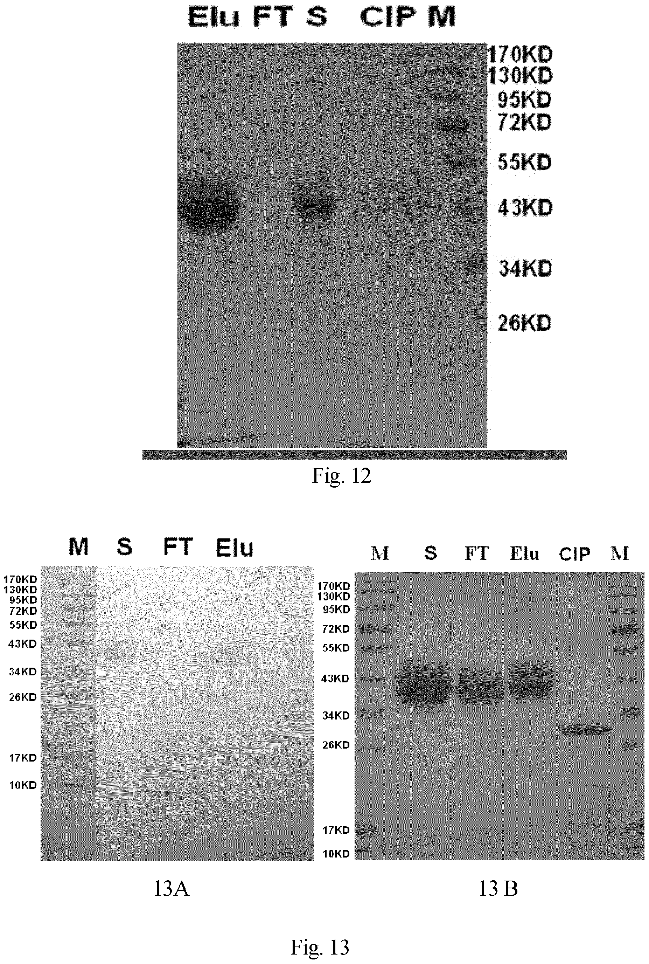

About 10 ml of Capto Adhere resin was packed onto the Econo-column 15/20 chromatography column. It was equilibrated with 200 ml of equilibration buffer (10mM phosphate buffer; pH 8.0) at a flow rate of 150 cm/h until the pH value and the conductivity were constant to baseline. The sample with the conductivity of 3.0ms/cm and pH of 6.9 was loaded. The sample was eluted with the elution buffer (46mMPB, 400mMNaCl, pH6.8) at a flow rate of 150cm/h. OsrAAT-containing fraction was collected. The electrophoretogram is shown in Figure 12.

### 4.2 Affinity Chromatography as Final Purification

### 4.2.1 Affinity Chromatography practiced by AAT-Select

About 20 ml of AAT Selectresin was packed onto the XK16/20 chromatography column. It was equilibrated with 200 ml of equilibration buffer (20 mMTris, 150mMNaCl, pH 7.4) at a flow rate of 150 cm/h until the pH value and the conductivity were constant to baseline. The sample with the conductivity of 10.9 ms/cm and pH of 6.9 was loaded. The sample was eluted using the elution buffer (20 mMTris, 2M MgCl₂, pH 7.4) at a flow rate of 150 cm/h. OsrAAT-containing fraction was collected. The electrophoretogram is shown in Figure13A.

### 4.2.2 Affinity Chromatography Practiced by ConASepharose FF 6B

About 10 ml of ConASepharose FF 6B resin was packed onto the XK16/20 chromatography column. It was equilibrated with 200 ml of equilibration buffer (20mMTris-HCl pH 7.4, 0.5M NaCl, 1mM Mn²⁺, 1mM Ca²⁺) at a flow rate of 150 cm/h until the pH value and the conductivity were constant to baseline. The sample with the conductivity of 10.9ms/cm and pH of 6.9 was loaded. The sample was eluted with the elution buffer (0.1M glucose) at a flow rate of 150cm/h. OsrAAT-containing fraction was collected. The electrophoretogram is shown in Figure 13B.

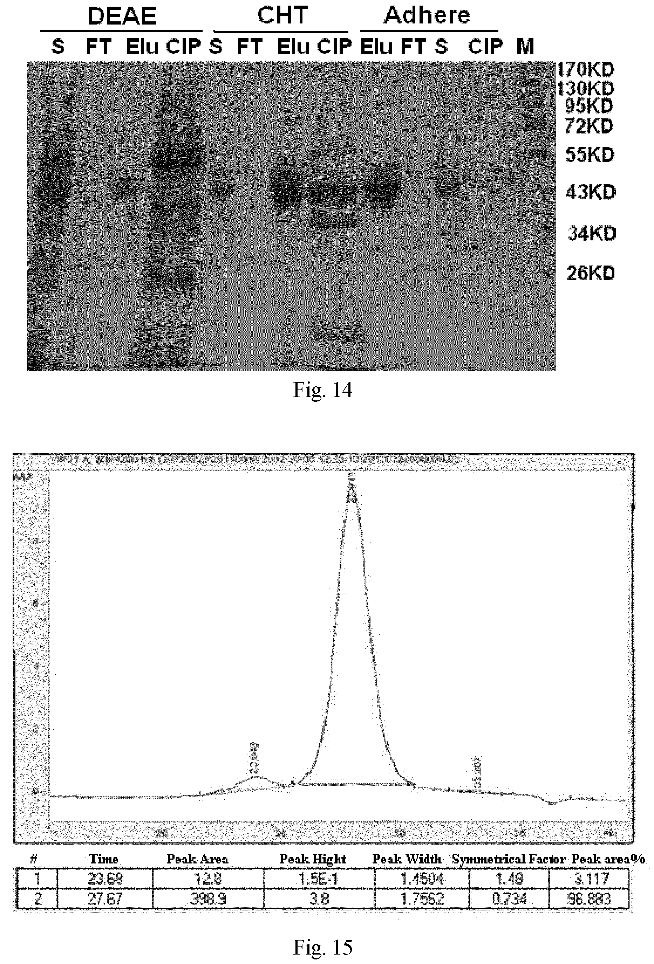

Taken together, the electrophoretograms from the primary, secondary and final purification steps are shown in Figure 14. The resulting OsrAAT was detected by HPLC, showing that the purity of OsrAAT was 97% by HPLC, as shown in Figure 15. As shown in Table 1,the recovery of OsrAAT reached up to 18.89±3.19%, corresponding to 0.336 g OsrAAT per kilogram brown rice.

**Table 1 Protein Recovery of Each Purification Step**

| Purification step | Total volume (ml) | Total protein content(mg) | Total antitrypsin (mg) | Reovery (%) |
|---|---|---|---|---|
| Extract | 1840 | 3078 | 360 | 100 |
| DEAE column chromatography | 660 | 429 | 189 | 52.5 |
| CHT column chromatography | 255 | 135 | 99 | 27.5 |
| Capto Adherecolumn chromatography | 130 | 68 | 68 | 18.9 |

### [Example 4] Biological Activity Assay of OsrAAT

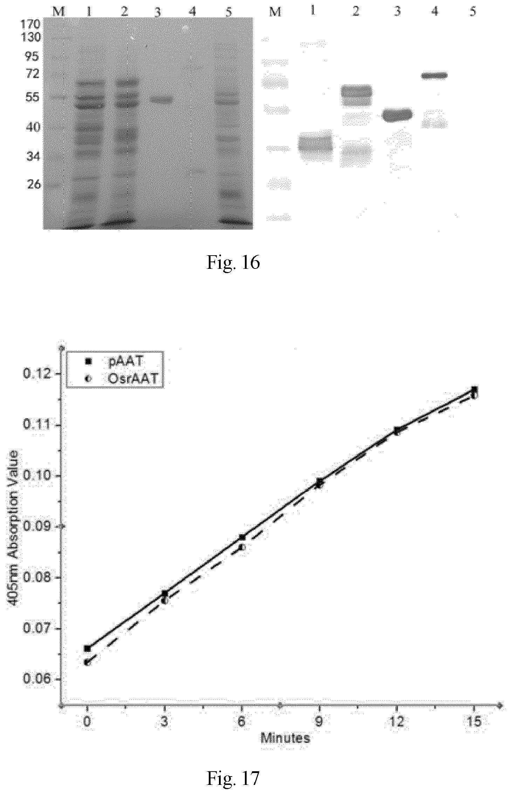

Band shift and porcine elastase inhibitory activity method (Huang et al. ) were used to assay the biological activity of OsrAAT that was expressed in rice endosperm. It was found from the results of band shift assay that the band was shifted, which was the complex covalently bound to porcine elastase when the crude protein extract was used, consisting with the results of Western blotting of plasma-derived AAT (pAAT). As shown in Figure 16, the result demonstrated that OsrAAT can effectively bind to specific substrate. In order to confirm whether OsrAAT has the same porcine elastase inhibition activity as pAAT, porcine elastase inhibitory activity method was performed. As shown in Figure 17, the results showed that the porcine elastase inhibitory activity of OsrAAT was identical to that of pAAT.

### References:

Agarwal, S., R. Singh, et al. (2008). "Expression of modified gene encoding functional human alpha-1-antitrypsin protein in transgenic tomato plants." Transgenic Res 17(5): 881-896.
Archibald, A. L., M. McClenaghan, et al. (1990). "High-level expression of biologically active human alpha 1-antitrypsin in the milk of transgenic mice." Proc. NatAcadSci, USA 87(13): 5178.
Blank, C. A. and M. Brantly (1994). "Clinical features and molecular characteristics of alpha 1-antitrypsin deficiency." Annals of allergy 72(2): 105.
Bollen, A., A. Herzog, et al. (1983). "Cloning and expression in Escherichia coli of full-length complementary DNA coding for human α1-antitrypsin." DNA 2(4): 255-264.
Brown, W. M. (2006). "rAAt (dermatological) Arriva/ProMetic." CurrOpinMolTher8(1): 69-75.
Cabanes-Macheteau, M., A. C. Fitchette-Laine, et al. (1999). "N-Glycosylation of a mouse IgG expressed in transgenic tobacco plants." Glycobiology9(4): 365-372.
Carlson, J. A., B. B. Rogers, et al. (1989). "Accumulation of PiZ alpha 1-antitrypsin causes liver damage in transgenic mice." Journal of Clinical Investigation 83(4): 1183.
Carver, A., G. Wright, et al. (1992). "Expression of human α1 antitrypsin in transgenic sheep." Cytotechnology9(1): 77-84.
Casolaro, M., G. Fells, et al. (1987). "Augmentation of lung antineutrophilelastase capacity with recombinant human alpha-1-antitrypsin." Journal of Applied Physiology 63(5): 2015-2023.
Chen, M., X. Liu, et al. (2005). "Modification of plant N□glycans processing: The future of producing therapeutic protein by transgenic plants." Medicinal research reviews 25(3): 343-360.
Churg, A., J. Dai, et al. (2001). "Alpha-1-antitrypsin and a broad spectrum metalloprotease inhibitor, RS113456, have similar acute anti-inflammatory effects." Laboratoryinvestigation81(8): 1119-1131.
Cregg, J. M., J. L. Cereghino, et al. (2000). "Recombinant protein expression in Pichiapastoris." Molecular biotechnology 16(1): 23-52.
Dalrymple, M. A. and I. Garner (1998). "Genetically modified livestock for the production of human proteins in milk." Biotechnology & genetic engineering reviews 15: 33-49.
Eriksson, S. (1996). "A 30-year perspective on α1-antitrypsin deficiency." Chest 110(6 Supplement): 237S-242S.
Gemgross, T. U. (2004). "Advances in the production of human therapeutic proteins in yeasts and filamentous fungi." Nature biotechnology 22(11): 1409-1414.
He, Y., T. Ning, et al. (2011). "Large-scale production of functional human serum albumin from transgenic rice seeds." ProcNatlAcadSci USA 108(47): 19078-19083.
Henquet, M., L. Lehle, et al. (2008). "Identification of the gene encoding the alpha1,3-mannosyltransferase (ALG3) in Arabidopsis and characterization of downstream n-glycan processing." Plant Cell 20(6): 1652-1664.
Hercz, A. (1985). "Proteolytic cleavages in [alpha] 1-antitrypsin and microheterogeneity." Biochemical and biophysical research communications 128(1): 199-203.
Heresi, G. A. and J. K. Stoller (2008). "Augmentation therapy in α-1 antitrypsin deficiency."
Huang, J., T. D. Sutliff, et al. (2001). "Expression and Purification of Functional Human α□1□Antitrypsin from Cultured Plant Cells." Biotechnology progress 17(1): 126-133.
Jones, A. M. and J. M. Helm (2009). "Emerging treatments in cystic fibrosis." Drugs 69(14): 1903-1910.
Karnaukhova, E. "Recent Advances in the Research and Development of Alpha-1 Proteinase Inhibitor for Therapeutic Use."
Karnaukhova, E., Y. Ophir, et al. (2006). "Recombinant human alpha-1 proteinase inhibitor: towards therapeutic use." Amino Acids 30(4): 317-332.
Karnaukhova, E., Y. Ophir, et al. (2004). Recombinant human alpha-1-proteinase inhibitor: glycosylation, stability and biological activity.
Krasnewich, D. M., G. D. Holt, et al. (1995). "Abnormal synthesisofdolichol-linkedoligosaccharides in carbohydrate-deficientglycoproteinsyndrome." Glycobiology5(5): 503-510.
Lerouge, P., M. Cabanes-Macheteau, et al. (1998). "N-glycoprotein biosynthesis in plants: recent developments and future trends." Plant Mol Biol38(1-2): 31-48.
Lewis, E. C., L. Shapiro, et al. (2005). "Alpha1-antitrypsin monotherapy prolongs islet allograft survival in mice." ProcNatlAcadSci U S A 102(34): 12153-12158.
Maras, M., I. van Die, et al. (1999). "Filamentous fungi as production organisms for glycoproteins of bio-medical interest." Glycoconjugate journal 16(2): 99-107.
Massoud, M., R. Bischoff, et al. (1991). "Expression of active recombinant human [alpha] 1-antitrypsin in transgenic rabbits." Journal of biotechnology 18(3): 193-204.
Mega, T., E. Lujan, et al. (1980). "Studies on the oligosaccharide chains of human alpha 1-protease inhibitor. II. Structure of oligosaccharides." Journal of Biological Chemistry 255(9): 4057-4061.
Nadai, M., J. Bally, et al. (2009). "High-level expression of active human alpha1-antitrypsin in transgenic tobacco chloroplasts." TransgenicRes18(2): 173-183.
Nevalainen, K., V. S. J. Te'o, et al. (2005). "Heterologousprotein expression in filamentousfungi." TRENDS in Biotechnology 23(9): 468-474.
Ning, T., T. Xie, et al. (2008). "Oral administration of recombinant human granulocyte-macrophage colony stimulating factor expressed in rice endosperm can increase leukocytes in mice." BiotechnolLett30(9): 1679-1686.
Rayon, C., M. Cabanes-Macheteau, et al. (1999). "Characterization of N-glycans from Arabidopsis. Application to a fucose-deficient mutant." Plant Physiol119(2): 725-734.
Shimada, T., M. Kuroyanagi, et al. (1997). "A pumpkin 72-kDa membrane protein of precursor-accumulating vesicles has characteristics of a vacuolar sorting receptor." Plant and cell physiology 38(12): 1414-1420.
Shimada, T., E. Watanabe, et al. (2002). "A vacuolar sorting receptor PV72 on the membrane of vesicles that accumulate precursors of seed storage proteins (PAC vesicles)." Plant and cell physiology 43(10): 1086-1095.
Spencer, L. T., J. E. Humphries, et al. (2005). "Antibody response to aerosolized transgenic human alpha1-antitrypsin." New England Journal of Medicine 352(19): 2030-2031.
Suzuki, Y. A., S. L. Kelleher, et al. (2003). "Expression, characterization, and biologic activity of recombinant human lactoferrin in rice." J PediatrGastroenterolNutr36(2): 190-199.
Tamer, I. M. and Y. Chisti (2001). "Production and recovery of recombinant protease inhibitor [alpha] 1-antitrypsin." Enzyme and microbial technology 29(10): 611-620.
Terashima, M., Y. Murai, et al. (1999). "Production of functional human alpha 1-antitrypsin by plant cell culture." ApplMicrobiolBiotechnol52(4): 516-523.
Vaughan, L., M. A. Lorier, et al. (1982). "[alpha] 1-antitrypsin microheterogeneity:: Isolation and physiological significance of isoforms." Biochimica et BiophysicaActa (BBA)-Protein Structure and Molecular Enzymology 701(3): 339-345.
Ward, M., C. Lin, et al. (2004). "Characterization of humanized antibodies secreted by Aspergillusniger." Applied and environmental microbiology 70(5): 2567-2576.
Wright, G., A. Carver, et al. (1991). "High level expression of active human alpha-1-antitrypsin in the milk of transgenic sheep." Nature biotechnology9(9): 830-834.
Xie, T., Q. Qiu, et al. (2008). "A biologically active rhIGF-1 fusion accumulated in transgenic rice seeds can reduce blood glucose in diabetic mice via oral delivery." Peptides 29(11): 1862-1870.
Yang, D., F. Guo, et al. (2003). "Expression and localization of human lysozyme in the endosperm of transgenic rice." Planta216(4): 597-603.
Ziomek, C. (1998). "Commercialization of proteins produced in the mammary gland." Theriogenology49(1): 139-144.

## Claims

1. Amethod of isolating and purifying rice genetic codon-optimized recombinant human antitrypsin (OsrAAT) from transgenic rice seeds containing OsrAAT, comprising the steps of:
a) preparing OsrAAT extract from the transgenic rice seeds containing OsrAAT as raw material;
b) subjecting the OsrAAT extract to anion exchange chromatography as a primary purification, to obtain primary OsrAAT elution fraction;
c) subjecting the primary OsrAAT elution fraction to composite chromatography of cation exchange with metal chelation chromatography as a secondary purification, to obtain secondary OsrAAT elution fraction;
d) subjecting the secondary OsrAAT elution fraction to composite chromatography of anion exchange with hydrophobic chromatography as a final purification, to obtain purified OsrAAT.

2. The method of claim 1, wherein it comprises the following steps of:
a) using transgenic rice seeds containing OsrAAT as raw material, hulling the paddy rice into brown rice and grinding it into milled rice with a fitness of 80-100 mesh; mixing the milled rice with an extraction buffer in a weight/volume ratio of 1:5-1:10 and extracting for 1 hour at room temperature; subjecting the resultant mixture to pressure filtration with a filter-cloth-type plate-and-frame filter press to obtain clear OsrAAT extract; wherein the components of the extraction buffer are: 20-25mM phosphate buffer, I-4mM mercaptoethanol, pH 6.9-7.1;
b) performing primary purification on a DEAE Sepharose FF chromatography column, equilibrating the column with 8-12 column volumes of pH 6.9-7.1, 20-25mM phosphate buffer at a flow rate of 100-180cm/h; using the OsrAAT extract of step 1 as a loading sample, wherein the sample having a conductivity of 2-3.5ms/cm and a pH of 6.8-7.0; eluting the sample with pH 6.8-7.1, 100-110mM PB buffer at a flow rate of 100-180cm/h, and collecting the elution fraction containing OsrAAT, to obtain primary OsrAAT elution fraction;
c) performing secondary purification on a cation exchange chromatography column with metal chelation capacity, equilibrating the column with 8-12 column volumes of pH 6.9-7.2, 5-12mM phosphate buffer at a flow rate of 100-150cm/h; diluting the primary OsrAAT elution fraction of step 2 to four times its original volume as a loading sample, wherein the sample having a conductivity of 2-3.5ms/cm and a pH of 6.8-7.0; eluting the sample with pH 6.8-7.1, 100-110mM PB buffer at a flow rate of 100-180cm/h, and collecting the elution fraction containing OsrAAT, to obtain secondary OsrAAT elution fraction;
d) performing final purification on a chromatography oolumn with cation exchange and hydrophobic characteristics, equilibrating the column with 8-12 column volumes of pH 7.5-8.2, 8-12mM phosphate buffer at a flow rate of 100-180cm/h; using the secondary OsrAAT elution fraction as a loading sample, wherein the sample having a conductivity of 2-3.5ms/cm and a pH of 6.8-7.1; eluting the sample with pH 6.6-7.0, 46mM PB, 400mM NaCl buffer at a flow rate of 100-180cm/h, and collecting the OsrAAT elution fraction containing OsrAAT, to obtain purified OsrAAT.

3. The method of claim 2, wherein it comprises the following steps of:
a) using transgenic rice seeds containing *OsrAAT* as raw material, hulling the paddy rice into half polished rice and grinding it into milled rice with a fitness of 80-100 mesh, mixing the milled rice with an extraction buffer in a weight/volume ratio of 1:10 and extracting for 1 hour at room temperature, subjecting the resultant mixture to pressure filtration with a filter-cloth-type plate-and-frame filter presser to obtain clear OsrAAT extract; wherein the componentsof the extraction buffer are: 20 mM phosphate buffer, I mM mercaptoethanol, pH 7.0;
b) performing primary purification on a DEAE Sepharose FF chromatography column, equilibrating the column with 10 oolumn volumes of pH 7.0,20 mM phosphate buffer at a flow rate of 150 cm/h; using the OsrAAT extract of step 1 as a loading sample, wherein the sample having a conductivity of 2.6 ms/cm and a pH of 6.95; eluting the sample with pH 7.0,108 mM PB buffer at a flow rate of 150cm/h, and collecting the elution fraction containing OsrAAT, to obtain primary OsrAAT elution fraction;
c) performing secondary purification on a cation exchange chromatography column with metal chelation capacity, equilibrating the column with 10 column volumes of pH 7.0, 10mM phosphate buffer at a flow rate of 150cm/h; diluting the primary OsrAAT elution fraction of step 2 to four times its original volume as a loading sample, wherein the sample having a conductivity of 3.0 ms/cm and a pH of 6.9; eluting the sample with pH 7.0, 108 mM PB buffer at a flow rate of 150 cm/h, and collecting the elution fraction containing OsrAAT, to obtain secondary OsrAAT elution fraction;
d) performing final purification on a chromatography oolumn with cation exchange and hydrophobic characteristics, equilibrating the column with 10 column volumes of pH 8.0, 10 mM phosphate buffer at a flow rate of 150 cm/h; using the secondary OsrAAT elution fraction as a loading sample, wherein the sample having a conductivity of 3.0 ms/cm and a pH of 6.9; eluting the sample with pH 6.8, 46 mM PB, 400 mM NaCl buffer at a flow rate of 150 cm/h, and collecting the elution fraction containing OsrAAT, to obtain purified OsrAAT

## Patentansprüche

1. Verfahren zur Isolierung und Reinigung von mit Reis genetisch codonoptimierter rekombinanter humaner Antitryptase (OsrAAT) aus transgenen Reissamen, die OsrAAT enthalten, umfassend die folgenden Schritte:
a) Herstellen eines OsrAAT-Extrakts aus den transgenen Reissamen, die OsrAAT enthalten, als Rohmaterial;
b) Unterwerfen des OsrAAT-Extrakts einer Anionenaustauschchromatographie als eine primäre Reinigung, um eine primäre OsrAAT-Elutionsfraktion zu erhalten;
c) Unterwerfen der primären OsrAAT-Elutionsfraktion einer Komposit-Chromatographie des Kationenaustauschs mit Metallchelatchromatographie als eine sekundäre Reinigung, um eine sekundäre OsrAAT-Elutionsfraktion zu erhalten;
d) Unterwerfen der sekundären OsrAAT-Elutionsfraktion einer Komposit-Chromatographie des Anionenaustauschs mit hydrophober Chromatographie als eine abschließende Reinigung, um gereinigte OsrAAT zu erhalten.

2. Verfahren nach Anspruch 1, wobei dieses die Folgenden Schritte umfasst:
a) Verwenden transgener Reissamen, die OsrAAT enthalten, als Rohmaterial, Enthüllen des Rohreises in braunen Reis und Zerkleinern von diesem in gemahlenen Reis mit einer Fitness von 80-100 Mesh; Mischen des zerkleinerten Reises mit einem Extraktionspuffer in einem Gewichts-/Volumen-Verhältnis von 1:5-1:10 und Extrahieren für 1 Stunde bei Raumtemperatur; Unterwerfen des resultierenden Gemisches einer Druckfiltration mit einer Platten-und-Rahmen-Filterpresse der Filter-GewebeArt, um ein klares OsrAAT-Extrakt zu erhalten; wobei die Komponenten des Extraktionspuffers Folgende sind: 20-25 mM Phosphatpuffer, 1-4 mM Merkaptoethylalkohol, ein pH-Wert von 6,9-7,1;
b) Durchführen einer primären Reinigung an einer DEAE-Sepharose-FF-Chromatographiesäule, Äquilibrieren der Säule mit 8-12 Säulenvolumen von 20-25 mM Phosphatpuffer mit einem pH-Wert von 6,9-7,1 bei einer Strömungsrate von 100-180 cm/h; Verwenden des OsrAAT-Extrakts aus Schritt 1 als eine Ladeprobe, wobei die Probe eine Leitfähigkeit von 2-3,5 ms/cm und einen pH-Wert von 6,8-7,0 aufweist; Eluieren der Probe mit 100-110 mM PB-Puffer mit einem pH-Wert von 6,8-7,1 bei einer Strömungsrate von 100-180 cm/h und Sammeln der Elutionsfraktion, die OsrAAT enthält, um eine primäre OsrAAT-Elutionsfraktion zu erhalten;
c) Durchführen einer sekundären Reinigung an einer Kationenaustausch-Chromatographiesäule mit Metallchelationkapazität, Äquilibrieren der Säule mit 8-12 Säulenvolumen von 5-12 mM Phosphatpuffer mit einem pH-Wert von 6,9-7,2 bei einer Strömungsrate von 100-150 cm/h; Verdünnen der primären OsrAAT-Elutionsfraktion aus Schritt 2 zu dem Vierfachen von deren ursprünglichem Volumen als eine Ladeprobe, wobei die Ladeprobe eine Leitfähigkeit von 2-3,5 ms/cm und einen pH-Wert von 6,8-7,0 aufweist; Eluieren der Probe mit 100-110 mM PB-Puffer mit einem pH-Wert von 6,8-7,1 bei einer Strömungsrate von 100-180 cm/h und Sammeln der Elutionsfraktion, die OsrAAT enthält, um eine sekundäre OsrAAT-Elutionsfraktion zu erhalten;
d) Durchführen einer abschließenden Reinigung an einer Chromatographiesäule mit Kationenaustausch und hydrophoben Eigenschaften, Äquilibrieren der Säule mit 8-12 Säulenvolumen von 8-12 mM Phosphatpuffer mit einem pH-Wert von 7,5-8,2 bei einer Strömungsrate von 100-180 cm/h; Verwenden der sekundären OsrAAT-Elutionsfraktion als eine Ladeprobe, wobei die Ladeprobe eine Leitfähigkeit von 2-3,5 ms/cm und einen pH-Wert von 6,8-7,1 aufweist; Eluieren der Probe mit 46 mM PB und 400 mM NaCl-Puffer mit einem pH-Wert von 6,6-7,0 bei einer Strömungsrate von 100-180 cm/h und Sammeln der OsrAAT Elutionsfraktion, die OsrAAT enthält, um gereinigte OsrAAT zu erhalten.

3. Verfahren nach Anspruch 2, wobei dieses die folgenden Schritte umfasst:
a) Verwenden von transgenen Reissamen, die OsrAAT enthalten, als Rohmaterial, Enthüllen des Rohreises in halbpolierten Reis und Zerkleinern von diesem in gemahlenen Reis mit einer Fitness von 80-100 Mesh, Mischen des zerkleinerten Reises mit einem Extraktionspuffer in einem Gewichts-/Volumen-Verhältnis von 1:10 und Extrahieren für 1 Stunde bei Raumtemperatur, Unterwerfen des resultierenden Gemisches einer Druckfiltration mit einer Platten-und-Rahmen-Filterpresse der Filter-GewebeArt, um ein klares OsrAAT-Extrakt zu erhalten; wobei die Komponenten des Extraktionspuffers Folgende sind: 20 mM Phosphatpuffer, 1 mM Merkaptoethylalkohol, ein pH-Wert von 7,0;
b) Durchführen einer primären Reinigung an einer DEAE-Sepharose-FF-Chromatographiesäule, Äquilibrieren der Säule mit 10 Säulenvolumen von 20 mM Phosphatpuffer mit einem pH-Wert von 7,0 bei einer Strömungsrate von 150 cm/h; Verwenden des OsrAAT-Extrakts aus Schritt 1 als eine Ladeprobe, wobei die Probe eine Leitfähigkeit von 2,6 ms/cm und einen pH-Wert von 6,95 aufweist; Eluieren der Probe mit 108 mM PB-Puffer mit einem pH-Wert von 7,0 bei einer Strömungsrate von 150 cm/h und Sammeln der Elutionsfraktion, die OsrAAT enthält, um eine primäre OsrAAT-Elutionsfraktion zu erhalten;
c) Durchführen einer sekundären Reinigung an einer Kationenaustausch-Chromatographiesäule mit Metallchelationkapazität, Äquilibrieren der Säule mit 10 Säulenvolumen von 10 mM Phosphatpuffer mit einem pH-Wert von 7,0 bei einer Strömungsrate von 150 cm/h; Verdünnen der primären OsrAAT-Elutionsfraktion aus Schritt 2 zu dem Vierfachen von deren ursprünglichem Volumen als eine Ladeprobe, wobei die Ladeprobe eine Leitfähigkeit von 3,0 ms/cm und einen pH-Wert von 6,9 aufweist; Eluieren der Probe mit 108 mM PB-Puffer mit einem pH-Wert von 7,0 bei einer Strömungsrate von 150 cm/h und Sammeln der Elutionsfraktion, die OsrAAT enthält, um eine sekundäre OsrAAT-Elutionsfraktion zu erhalten;
d) Durchführen einer abschließenden Reinigung an einer Chromatographiesäule mit Kationenaustausch und hydrophoben Eigenschaften, Äquilibrieren der Säule mit 10 Säulenvolumen von 10 mM Phosphatpuffer mit einem pH-Wert von 8,0 bei einer Strömungsrate von 150 cm/h; Verwenden der sekundären OsrAAT-Elutionsfraktion als eine Ladeprobe, wobei die Ladeprobe eine Leitfähigkeit von 3,0 ms/cm und einen pH-Wert von 6,9 aufweist; Eluieren der Probe mit 46 mM Pb und 400 mM NaCl-Puffer mit einem pH-Wert von 6,8 bei einer Strömungsrate von 150 cm/h und Sammeln der Elutionsfraktion, die OsrAAT enthält, um eine gereinigte OsrAAT-Elutionsfraktion zu erhalten.

## Revendications

1. Procédé d'isolation et de purification de l'antitrypsine humaine recombinante de riz optimisée pour l'usage du code génétique, (OsrAAT) à partir de semences de riz transgénique contenant de l'OsrAAT, comprenant les étapes :
a) de préparation d'un extrait d'OsrAAT à partir des semences de riz transgénique contenant de l'OsrAAT en tant que matière première ;
b) de soumission de l'extrait d'OsrAAT à une chromatographie par échange d'anions en tant que purification primaire, pour obtenir une fraction d'élution d'OsrAAT primaire ;
c) de soumission de la fraction d'élution d'OsrAAT primaire à une chromatographie composite d'échange de cations avec une chromatographie par chélation métallique en tant que purification secondaire, pour obtenir une fraction d'élution d'OsrAAT secondaire ;
d) de soumission de la fraction d'élution d'OsrAAT secondaire à une chromatographie composite d'échange d'anions avec une chromatographie hydrophobe en tant que purification finale, pour obtenir de l'OsrAAT purifié.

2. Procédé selon la revendication 1, dans lequel il comprend les étapes suivantes :
a) d'utilisation de semences de riz transgénique contenant de l'OsrAAT en tant que matière première, en décortiquant le riz paddy en riz brun et en le broyant en riz blanchi avec une qualité de 80 à 100 mesh ; de mélange du riz blanchi avec un tampon d'extraction dans un rapport poids/volume de 1:5 à 1:10 et d'extraction pendant 1 heure à température ambiante ; de soumission du mélange résultant à une filtration sous pression avec un filtre-presse à plaques et cadres de type linge de filtration pour obtenir un extrait d'OsrAAT pure ; dans lequel les composants du tampon d'extraction sont : un tampon phosphate 20 à 25 mM, du mercaptoéthanol 1 à 4 mM, un pH de 6,9 à 7,1 ;
b) de mise en oeuvre d'une purification primaire sur une colonne de chromatographie FF sur DEAE Sépharose, en équilibrant la colonne avec 8 à 12 volumes de colonne d'un tampon phosphate 20 à 25 mM de pH de 6,9 à 7,1, à un débit de 100 à 180 cm/h ; d'utilisation de l'extrait d'OsrAAT de l'étape 1 en tant qu'échantillon de chargement, dans lequel l'échantillon a une conductivité de 2 à 3,5 ms/cm et un pH de 6,8 à 7,0 ; d'élution de l'échantillon avec un tampon PB 100 à 110 mM avec un pH de 6,8 à 7,1 à un débit de 100 à 180 cm/h, et de collecte de la fraction d'élution contenant de l'OsrAAT, pour obtenir une fraction d'élution d'OsrAAT primaire ;
c) de mise en oeuvre d'une purification secondaire sur une colonne de chromatographie d'échange de cation avec une capacité de chélation métallique, en équilibrant la colonne avec 8 à 12 volumes de colonne d'un tampon phosphate 5 à 12 mM de pH de 6,9 à 7,2 à un débit de 100 à 150 cm/h ; de dilution de la fraction d'élution d'OsrAAT primaire de l'étape 2 en quatre fois son volume d'origine en tant qu'échantillon de chargement, dans lequel l'échantillon a une conductivité de 2 à 3,5 ms/cm et un pH de 6,8 à 7,0 ; d'élution de l'échantillon avec un tampon PB 100 à 110 mM de pH de 6,8 à 7,1 à un débit de 100 à 180 cm/h, et de collecte de la fraction d'élution contenant de l'OsrAAT, pour obtenir une fraction d'élution d'OsrAAT secondaire ;
d) de mise en oeuvre d'une purification finale sur une colonne de chromatographie avec des caractéristiques d'échange de cations et hydrophobes, en équilibrant la colonne avec 8 à 12 volumes de colonne d'un tampon phosphate 8 à 12 mM de pH de 7,5 à 8,2 à un débit de 100 à 180 cm/h ; d'utilisation de la fraction d'élution d'OsrAAT secondaire en tant qu'échantillon de chargement, dans lequel l'échantillon a une conductivité de 2 à 3,5 ms/cm et un pH de 6,8 à 7,1 ; d'élution de l'échantillon avec un tampon PB 46 mM, NaCl 400 mM, de pH de 6,6 à 7,0, à un débit de 100 à 180 cm/h, et de collecte de la fraction d'élution d'OsrAAT contenant de l'OsrAAT, pour obtenir de l'OsrAAT purifiée.

3. Procédé selon la revendication 2, dans lequel il comprend les étapes suivantes :
a) d'utilisation de semences de riz transgénique contenant de l'OsrAAT en tant que matière première, en décortiquant le riz paddy en riz à moitié poli et en le broyant en riz blanchi avec une qualité de 80 à 100 mesh, de mélange du riz broyé avec un tampon d'extraction dans un rapport poids/volume de 1:10 et d'extraction pendant 1 heure à température ambiante, de soumission du mélange résultant à une filtration sous pression avec un filtre-presse à plaques et cadres de type linge de filtration pour obtenir un extrait d'OsrAAT pure ; dans lequel les composants du tampon d'extraction sont : un tampon phosphate 20 mM, du mercaptoéthanol 1 mM, un pH de 7,0 ;
b) de mise en oeuvre d'une purification primaire sur une colonne de chromatographie FF sur DEAE Sépharose, en équilibrant la colonne avec 10 volumes de colonne avec un tampon phosphate 20 mM avec un pH de 7,0 à un débit de 150 cm/h ; d'utilisation de l'extrait d'OsrAAT de l'étape 1 en tant qu'échantillon de chargement, dans lequel l'échantillon a une conductivité de 2,6 ms/cm et un pH de 6,95 ; d'élution de l'échantillon avec un tampon PB 108 mM avec un pH de 7,0 à un débit de 150 cm/h, et de collecte de la fraction d'élution contenant de l'OsrAAT, pour obtenir une fraction d'élution d'OsrAAT primaire ;
c) de mise en oeuvre d'une purification secondaire sur une colonne de chromatographie d'échange de cations avec capacité de chélation métallique, en équilibrant la colonne avec 10 volumes de colonne avec un tampon phosphate 10 mM de pH 7,0 à un débit de 150 cm/h ; de dilution de la fraction d'élution d'OsrAAT primaire de l'étape 2 en quatre fois son volume d'origine en tant qu'échantillon de chargement, dans lequel l'échantillon a une conductivité de 3,0 ms/cm et un pH de 6,9 ; d'élution de l'échantillon avec un tampon PB 108 mM avec un pH de 7,0 à un débit de 150 cm/h, et de collecte de la fraction d'élution contenant de l'OsrAAT, pour obtenir une fraction d'élution d'OsrAAT secondaire ;
d) de mise en oeuvre d'une purification finale sur une colonne de chromatographie avec des caractéristiques d'échange de cations et hydrophobes, en équilibrant la colonne avec 10 volumes de colonne avec un tampon phosphate 10 mM de pH 8,0 à un débit de 150 cm/h ; d'utilisation de la fraction d'élution d'OsrAAT secondaire en tant qu'échantillon de chargement, dans lequel l'échantillon a une conductivité de 3,0 ms/cm et un pH de 6,9 ; d'élution de l'échantillon avec un tampon PB 46 mM, NaCl 400 mM de pH 6,8 à un débit de 150 cm/h, et de collecte de la fraction d'élution contenant de l'OsrAAT, pour obtenir de l'OsrAAT purifiée.
